# EUROPÄISCHE PATENTSCHRIFT

(11) **EP 1 137 623 B1**
(45) Veröffentlichungstag und Bekanntmachung des Hinweises auf die Patenterteilung: **04.06.2003**
(21) Anmeldenummer: 99963444.7
(22) Anmeldetag: 09.12.1999
(51) Int. Cl.: C07C 209/32, C07C 209/34, C07C 209/36, B01J 19/24, B01J 19/26, B01J 10/00

(54) **VERFAHREN ZUR HERSTELLUNG VON AMINEN**
METHOD FOR PRODUCING AMINES
PROCEDE POUR LA PRODUCTION D'AMINES

(30) Priorität: 12.12.1998 DE 19857409; 14.07.1999 DE 19932821
(43) Veröffentlichungstag der Anmeldung: 04.10.2001
(73) Patentinhaber: BASF AKTIENGESELLSCHAFT, 67056 Ludwigshafen (DE)
(72) Erfinder: SANDER, Michael, D-01945 Ruhland (DE); PENZEL, Ulrich, D-01945 Tettau (DE); SCHWARZ, Hans, Volkmar, Baton Rouge, LA 70817 (US); STRÖFER, Eckhard, D-68163 Mannheim (DE); STÜTZER, Dieter, D-67373 Dudenhofen (DE); MÜLLER, Jörn, D-67071 Ludwigshafen (DE); MAURER, Markus, D-67071 Ludwigshafen (DE); ZEHNER, Peter, D-67074 Ludwigshafen (DE); SCHWAB, Ekkehard, D-67434 Neustadt (DE); BÖHLING, Ralf, D-64347 Griesheim (DE); VANOPPEN, Dominic, D-67136 Fussgönheim (DE)
(86) Internationale Anmeldenummer: EP9909680
(87) Internationale Veröffentlichungsnummer: WO00035852

(56) Entgegenhaltungen:
- EP-A- 0 124 010
- EP-A- 0 263 935
- EP-A- 0 634 391
- DE-A- 2 736 872
- GB-A- 1 452 466
- US-A- 2 823 235
- US-A- 3 636 152
- US-A- 4 212 824
- US-A- 4 482 696

## Beschreibung

Die Erfindung betrifft ein kontinuierliches Verfahren zur Herstellung von Aminen, insbesondere aromatischen Aminen, durch katalytische Hydrierung der den Aminen zugrunde liegenden Nitroverbindungen.

Die Herstellung von Aminen, insbesondere von aromatischen Monound/oder Polyaminen durch katalytische Hydrierung der entsprechenden Mono- und/oder Polynitroverbindungen ist seit langem bekannt und vielfach in der Literatur beschrieben.

Bei der in der Technik üblichen Herstellung der aromatischen Mono- und/oder Polyamine durch Umsetzung von Nitroverbindungen mit Wasserstoff wird eine beträchtliche Wärmemenge frei. Zumeist wird die Hydrierung daher in der Technik bei möglichst niedrigen Temperaturen unter Einsatz von Hydrierkatalysatoren in der Flüssigphase durchgeführt. Dabei wird die zu reduzierende Verbindung in einem Lösungsmittel mit dem Katalysator vermischt und diskontinuierlich in einem Autoklaven oder kontinuierlich in einem Schlaufenreaktor, einer Blasensäule oder einer Reaktorkaskade reduziert. Bei diesen bisher bekannten Verfahren gibt es eine Reihe von Nachteilen, wie z.B. die Notwendigkeit des Austragens und besonders des Ausschleusen desaktivierter Katalysatoranteile, was zu Katalysatorverlusten führt. Ferner stellen die häufig auftretenden Nebenreaktionen, die zur Bildung störender Substanzen, wie z.B. teerartiger Bestandteile, und damit zu Ausbeuteminderungen führen, ein Problem vieler bislang verwendeter Verfahren dar.

Um diese Nachteile zu verringern, ist es bekannt, den Katalysator in einem Festbett anzuordnen. So beschreibt DE-OS 2 135 154 die Hydrierung einer Nitroverbindung allein oder in Anwesenheit eines Verdünnungsmittel in flüssigem Zustand in einem Röhrenreaktor in Anwesenheit eines Palladium-Katalysators auf Spinell in einem Festbett. Bei der Verwendung dieses Palladium-Katalysators auf Spinell ist die Katalysatorherstellung sehr aufwendig, und eine gezielte Fixierung auf dem Träger nur teilweise möglich. Ferner führt diese Festbetthydrierung zu geringen Hydrierausbeuten und zur Bildung von hochsiedenden Nebenprodukten. Beispielhaft erwähnt seien in diesem Zusammenhang hydrogenolytische Spaltungen, Kernhydrierungen oder die Bildung von hochmolekularen, teerartigen Substanzen. Es können infolge des stark exothermen Reaktionsverlaufs der Nitrogruppenumsetzung und der hohen Reaktionsgeschwindigkeit bei höheren Temperaturen auch explosionsartige Nebenreaktionen auftreten.

Um diese unerwünschten Nebenreaktionen soweit wie möglich auszu-schließen, wurde daher im allgemeinen die großtechnische Hydrierung von aromatische Nitroverbindungen bei relativ niedrigen Temperaturen durchgeführt.

In EP-A-124 010 wird ein Verfahren zur Herstellung von aromati-schen Diaminen durch katalytische Hydrierung der entsprechenden Dinitroverbindungen unter gleichzeitiger Erzeugung von Dampf mit einem Überdruck von > 1 bar beschrieben. Als Reaktor wird eine mit Field-Rohren bestückte Blasensäule verwendet. Eine Reaktionssuspension, die im wesentlichen aus einer aromatischen Dinitro-Verbindung, dem entsprechenden Diamin, einem Hydrierungskatalysator, einem gesättigten, aliphatischen Alkohol mit 1 bis 6 Kohlenstoffatomen als Lösungsmittel und Wasser besteht, wird mit Wasserstoff in den Reaktor eingespeist. Die Menge der in der Blasensäule eingespeisten Reaktionssuspension sowie der Druck, die D Temperatur und die Menge an Kühlwasser werden so bemessen, daß in der Blasensäule eine Reaktionstemperatur zwischen 140 und 250 °C vorliegt. Als Katalysatoren werden bekannte Hydrierkatalysatoren verwendet, wobei vorzugsweise Metalle der VIII. Nebengruppe des Periodensystems und insbesondere Raney-Eisen, -Kobalt und -Nickel eingesetzt werden.

Nachteilig bei dem in EP-A-124 010 beschriebenen Verfahren ist die Verwendung großer Mengen an Lösungsmittel, die zwar die Probleme bei der Hydrierung von Polynitroverbindungen bei höheren Temperaturen mindern, unter den Hydrierungsbedingungen jedoch nicht vollständig inert sind, was zu unerwünschten Nebenprodukten und Ausbeuteverminderungen führt.

In EP-A-263 935 werden Rührkesselreaktoren zur Durchführung von exothermen Reaktionen beschrieben, die sich dadurch auszeichnen, daß die Kühlung der Reaktoren mittels Wasser erfolgt, das in Fieldrohren in Dampf überführt wird. Fieldrohr-Wärmetauscher sind durch ein hohes Verhältnis von Wärmetauscherfläche zu Volumen des Reaktionsraums gekennzeichnet und somit besonders effektiv in der Abfuhr freiwerdender Reaktionswärme. Das in EP-A-263 935 beschriebene Verfahren ist jedoch nur bedingt für katalytische Hydrierungen einsetzbar, da eine optimale Phasendurchmischung nicht sichergestellt ist. Insbesondere muß stets eine hohe Wasserstoffkonzentration über der Reaktionsmischung gewährleistet sein, um ein Nachlösen von Wasserstoff in der Reaktionsmischung zu gewährleisten. Trotzdem bilden sich im Reaktor Zonen mit partieller Verarmung an Wasserstoff aus. Aufgrund der zu erwartenden Inhomogenitäten laufen verstärkt unkontrollierbare Nebenreaktionen unter Ausbeuteverlusten ab. Die Kühlflächen belegen sich zudem mit hochmolekularen Verbindungen und/oder Katalysatoranteilen. Außerdem wird bei diesem Verfahren der Katalysator 5 mechanisch stark beansprucht, was zu einer verminderten Standzeit des Katalysators führt.

In EP-A-634 391 wird ein Verfahren zur Hydrierung von aromatischen Polynitroverbindungen zu Aminen beschrieben, in dem durch .0 technologische Optimierung unter Einsatz eines Loop-Venturi-Reaktors mit einem Ejektor, gekoppelt mit speziellen Bedingungen wie genauem Umwälzvolumenverhältnis, genauem Energieeintrag, einem genau eingestellten Wasserstoffvolumenstrom, die genannten Probleme der Hydrierung von aromatischen Polynitroverbindungen mini-5 miert werden sollen. Als Katalysatoren werden die in EP-A-124 010 beschriebenen Verbindungen verwendet.

Bei diesem Verfahren kann es, bedingt durch die Anordnung eines Wärmetauschers zur Abführung der Reaktionswärme außerhalb des Schlaufenreaktors, im Ejektor und im Reaktor zu örtlichen überhitzungen mit sofortigem Einsetzen von Nebenreaktionen wie Kernhydrierungen, hydrogenolytischen Spaltungen bzw. Bildung von hochmolekularen, teerartigen Produkten, die die Katalysatoroberfläche belegen, kommen. Darüber hinaus stellt sich im Reaktor-5 volumen außerhalb des Ejektors eine bezüglich des Strömungs- und Verweilzeitverhaltens reine Blasensäulencharakteristik ein, in dem regellose klein- und großräumige Wirbel mit vergleichsweise geringer Stoffübergangsleistung auftreten. Eine wesentliche Verbesserung der Hydrierausbeute, der Hydrierselektivität und der Raum-Zeit-Ausbeute wird somit bei diesem Verfahren kaum erreicht. Außerdem wird auch hier durch das Umpumpen der gesamten Reaktionsmischung der Katalysator mechanisch stark beansprucht, was wiederum zu einer verminderten Standzeit des Katalysators führt.

GB-A-1 452 466 beschreibt ein Verfahren zur Nitrobenzolhydrierung, bei dem ein adiabater Reaktor einem isothermen Reaktor nachgeschaltet ist. Dabei wird der größte Teil des Nitrobenzols in einem thermostatisierten Rohrbündelreaktor umgesetzt, lediglich die Hydrierung des Restgehaltes an Nitrobenzol erfolgt bei relativ geringem Wasserstoffüberschuß von kleiner 30:1 in einem adiabaten Reaktor. Dieses Verfahren ist jedoch technisch sehr aufwendig.

DE-B-1 809 711 befaßt sich mit dem gleichmäßigen Einbringen von flüssigen Nitroverbindungen in einen heißen Gasstrom durch Verdüsen, bevorzugt an verengten Stellen unmittelbar vor dem Reaktor. Dem Verfahren ist jedoch zu entnehmen, daß trotz eines beachtlichen Wasserstoffüberschusses ein großer Teil der Reaktionsenthalpie den Reaktor nicht mit dem Produktgas verlassen hat, was zu Kühlproblemen führt.

In DE-A-36 36 984 wird ein Verfahren zur gekoppelten Produktion von Nitro- und Dinitro-aromaten aus den entsprechenden aromatischen Kohlenwasserstoffen durch Nitrierung und deren nachfolgende Hydrierung beschrieben. Die Hydrierung erfolgt in der Gasphase bei Temperaturen zwischen 176 und 343,5 °C. Der Wasserstoffstrom 10 dient bei diesem Verfahren auch zur Ableitung der Reaktionswärme aus den Reaktoren. Die beschrieben Apparatur zur Gasphasenhydrierung, besteht im wesentlichen aus zwei hintereinander geschalteten Reaktoren mit Zwischenkühlung und Eduktzwischeneinspeisung, auf deren Größe jedoch nicht eingegangen wird. Dem 15 Temperaturprofil der Reaktoren kann man allerdings entnehmen, daß ein nicht unerheblicher Teil der Reaktionswärme auf Grund der geringen Wärmekapazität des Wasserstoffs nicht mit dem Produktgasstrom den Reaktor verläßt. Nachteilig an diesem Verfahren ist weiterhin das energieintensive Verdampfen der Nitroaromaten.

Aufgabe der Erfindung war es, ein apparativ einfaches kontinuierliches Verfahren zur Herstellung von Aminen, insbesondere aromatischen Aminen, durch Hydrierung von Nitroverbindungen, insbesondere aromatischen Nitroverbindungen in Gegenwart von Hydrierkatalysatoren, vorzugsweise geträgerten Metallkatalysatoren, unter Verbesserung der Raum-Zeit-Ausbeute und der Hydrierselektivität unter Vermeidung von örtlichen Überhitzungen und dadurch verstärkt ablaufenden Nebenreaktionen zu entwickeln.

Die Aufgabe konnte überraschenderweise gelöst werden durch ein Verfahren zur Herstellung von Aminen, insbesondere aromatischen Aminen, durch Hydrierung der entsprechenden Nitroverbindungen in einem vertikalen, vorzugsweise zylindrischen Reaktor, dessen Länge größer ist als sein Durchmesser, mit einer im oberen Bereich des Reaktors angeordneten, nach unten gerichteten Strahldüse. über die die Edukte sowie das Reaktionsgemisch zugeführt werden sowie mit einem Abzug an einer beliebigen Stelle des Reaktors, vorzugsweise im unteren Bereich, über den das Reaktionsgemisch in einem äußeren Kreislauf mittels eines Förderorgans, 0 vorzugsweise einer Pumpe, der Strahldüse wieder zugeführt wird, sowie einer Strömungsumkehr im unteren Bereich des Reaktors.

Gegenstand der Erfindung ist demzufolge ein Verfahren zur Herstellung von Aminen, insbesondere aromatischen Aminen, durch Hydrierung der entsprechenden Nitroaromaten, dadurch gekennzeichnet, daß die Umsetzung in einem vertikalen, vorzugsweise zylindrischen Reaktor, dessen Länge größer ist als sein Durchmesser, mit einer im oberen Bereich des Reaktors angeordneten, nach unten gerichteten Strahldüse, über die die Edukte sowie das Reaktionsgemisch zugeführt werden sowie mit einem Abzug an einer beliebigen Stelle des Reaktors, vorzugsweise im unteren Bereich, über 5 den das Reaktionsgemisch in einem äußeren Kreislauf mittels eines Förderorgans, vorzugsweise einer Pumpe, der Strahldüse wieder zugeführt wird, sowie einer Strömungsumkehr im unteren Bereich des Reaktors durchgeführt wird und im Reaktion integrierte Wärmetauscher angebracht sind. Der beschriebene Reaktor wird im folgenden als Schlaufenreaktor bezeichnet.

Die Strömungsumkehr und damit die Ausbildung einer internen Schlaufenströmung kann beim Abzug der Reaktionsmischung im oberen Bereich des Reaktors durch den Aufprall der eingedüsten Reaktionsmischung auf den Reaktorboden erfolgen. Bei dem bevorzugten 5 Abzug der Reaktionsmischung im unteren Bereich des Reaktors wird die Strömungsumkehr durch Einbauten, insbesondere einer zur Reaktorwand senkrechten Prallplatte, bewirkt.

In einer bevorzugten Ausgestaltung des erfindungsgemäßen Verfahrens weist der Schlaufenreaktor zwischen der Düse und der Strömungsumkehr ein vorzugsweise konzentrisches, zur Reaktorwand paralleles Einsteckrohr auf. Dieses Einsteckrohr kann als einfaches Rohr, als rohrförmiger Plattenwärmetauscher oder als spiralförmiges Kühlrohr ausgestaltet sein.

Durch das konzentrische Einsteckrohr wird in Verbindung mit der Prallplatte die Schlaufenströmung innerhalb des Reaktors, im folgenden als interne Schlaufenströmung bezeichnet, stabilisiert. Neben der Strömungsumkehr bewirkt die Prallplatte, daß keine Gasblasen in die externe Schlaufenströmung mitgerissen werden und die Pumpe beschädigen.

Dadurch, daß der Durchmesser des Schlaufenreaktors geringer ist als seine Höhe, wird eine ausreichende Schlaufenströmung im gesamten Reaktor bewirkt und die Ausbildung von Zonen ohne Strömung verhindert.

In einer weiteren bevorzugten Ausführungsform der Erfindung besitzt der Reaktor im Reaktorinnenraum integrierte Wärmetauscher. Diese Wärmetauscher sollen so gestaltet sein, daß sie die interne Schlaufenströmung nicht behindern und keine Turbulenzen hervorrufen. Als Wärmetauscher können beispielsweise mit Kühlmedium durchströmte Rohre, deren Richtung vorzugsweise parallel zur Reaktorwand verläuft, Plattenwärmetauscher, die vorzugsweise parallel zur Reaktorwandung verlaufen, oder auch unten geschlossene Siederohre, wie sie in EP-A-263 935 beschrieben sind, sogenannte Fieldrohre, eingesetzt werden. Bei der Verwendung von Fieldrohren ist es möglich, den entstehenden Dampf als Prozeßdampf zu nutzen.

In dieser Ausführungsform kann man den erfindungsgemäß verwende-5 ten Reaktor als Reaktionswärmetauscher bezeichnen, da die Reaktionswärme am Ort des Entstehens abgeführt wird. Es ist auch möglich, zusätzlich zu den im Reaktor integrierten Wärmetauschern einen Wärmetauscher in der externen Schlaufenströmung anzubringen.

Der überwiegende Teil des Reaktionsgemisches wird in der internen Schlaufenströmung geführt, lediglich ein geringer Anteil des Reaktionsgemisches wird extern umgepumpt und sorgt so für den Antrieb der Schlaufenströmung. Das Verhältnis der Volumenströme von interner Schlaufenströmung zu externer Schlaufenströmung beträgt 2:1 bis 30:1, bevorzugt 5:1 bis 10:1.

Durch den geringen Anteil der externen Schlaufenströmung an der gesamten Reaktionsmischung werden deutlich geringere Mengen an Katalysator pro Zeiteinheit über die Kreislaufpumpe umgewälzt als im Fall der Kühlung über einen externen Wärmetauscher. Dies führt zu einer Verringerung der mechanischen Katalysatorbeanspruchung und damit zu einer längeren Lebensdauer des Katalysators. Außerdem gewährleistet diese Ausführungsform in Verbindung mit den integrierten Wärmetauschern eine hohe Isothermie der Reaktion, d.h. einen sehr kleinen Temperaturgradienten über die Reaktorhöhe, da die Hydrierung praktisch vollständig in der internen Schlaufenströmung abläuft und die Reaktionswärme so bereits am Ort ihrer Entstehung abgeführt wird. Dabei werden Beschränkungen der Reaktionsgeschwindigkeit durch Stoff- und Wärmetransport praktisch vollständig ausgeschaltet. Nebenreaktionen, die durch Temperaturgradienten im Reaktionssystem begünstigt werden, werden praktisch vollständig unterdrückt. Die Reaktionsgeschwindigkeit ist dann nur noch durch die Reaktionskinetik begrenzt. Außerdem ist die Sicherheit des Verfahrens gegenüber einer Kühlung im äußeren Kreislauf verbessert, da die Reaktorkühlung auch dann noch funktioniert, wenn die Pumpe für den äußeren Kreislauf ausfällt.

Die Strahldüse kann als Ein- oder Zweistoffdüse ausgelegt werden. Bei der Einstoffdüse wird nur das flüssige Reaktionsgemisch eingedüst und der Wasserstoff an einer beliebigen anderen Stelle, vorzugsweise jedoch in der Flüssigphase, dem Reaktor zugeführt. Vorteilhaft bei dieser Ausgestaltung ist der einfache Aufbau dieser Düse, nachteilig ist die schlechtere Dispergierung des Wasserstoffs im Reaktionsgemisch. Bei der Zweistoffdüse, die im Aufbau aufwendiger ist, wird der Wasserstoff düsenmittig zugeführt und dispergiert. Bei dieser Ausgestaltung des Verfahrens ist die Dispergierung des Wasserstoffs in der Reaktionsmischung wesentlich besser. Es kann praktisch ausgeschlossen werden, daß es in einzelnen Zonen des Reaktors zu einer partiellen Verarmung an Wasserstoff kommt.

Im Reaktor wird vorzugsweise, unabhängig von der Art der eingesetzten Nitroverbindungen, ein Druck von 5 bis 100 bar, bevorzugt 10 bis 50 bar, und eine Betriebstemperatur von 80 bis 200 °C, bevorzugt 100 bis 150 °C, aufrechterhalten. Der Leistungseintrag beträgt vorzugsweise an der Düse 15 bis 30 kW/l im gesamten Reaktionssystem 3 bis 10 W/l.

Der Produktaustrag aus dem System erfolgt kontinuierlich an beliebiger Stelle. Bevorzugt erfolgt der Produktaustrag im unteren Reaktorbereich am Boden des Reaktors oder insbesondere aus der äußeren Schlaufenströmung über eine Katalysatorabtrenneinheit oder ohne eine solche. Diese Abtrenneinheit kann ein Schwerkraftabscheider, z. B. ein Settler, ein geeigneter Filter, beispielsweise ein Querstromfilter, oder eine Zentrifuge sein. Der Katalysator kann vom Produkt abgetrennt und anschließend in das Reaktorsystem zurückgeführt werden. Bevorzugt erfolgt der Produktaustrag unter Rückhaltung des Katalysators. Das Amin kann danach nach den üblichen und bekannten Verfahren, beispielsweise durch Destillation oder Extraktion, gereinigt werden.

Zwischen dem Abzug der Reaktionsmischung und der Abtrenneinheit kann sich zur Vervollsständigung der Umsetzung ein Nachreaktor befinden. Dieser kann wie ein erfindungsgemäßer Reaktor ausgestaltet sein, es kann auch mindestens ein Rührkessel und/oder Strömungsrohr verwendet werden.

Im erfindungsgemäßen Verfahren wird die Mono- und/oder Polynitroverbindung in reiner Form, als Mischung mit dem entsprechenden Mono- und/oder Polyamin, als Mischung mit dem entsprechenden Mono- und/oder Polyamin und Wasser oder als Mischung mit dem entsprechenden Mono- und/oder Polyamin, Wasser und einem insbesondere alkoholischen Lösungsmittel eingesetzt. Die aromatische Mono- und/oder Polynitroverbindung wird fein verteilt in das Gemisch eingetragen. Vorzugsweise wird die Nitroverbindung in die Strahldüse eingetragen, besonders bevorzugt in den Mischraum der Düse.

Vorzugsweise werden im Rahmen des erfindungsgemäßen Verfahrens aromatische Nitroverbindungen mit einer oder mehreren Nitrogruppen und 6 bis 18 C-Atomen, bsp. Nitrobenzole, wie z.B. Nitrobenzol, 1.3-Dinitrobenzol. Nitrotoluole, wie z.B. 2,4-, 2,6-Dinitrotoluol, 2,4,6-Trinitrotoluol, Nitroxylole, wie z.B. 1,2-Dimethyl-3-, 1,2 Dimethyl-4-, 1,4-Dimethyl-2-, 1,3-Dimethyl-2-, 2,4-Dimethyl-2- und 1, 3-Dimethyl-5-nitrobenzol, Nitronaphthaline, wie z.B. 1-, 2-Nitronaphthalin, 1,5 und 1,8-Dinitronaphthalin, Chlornitrobenzole, wie z.B. 2-Chlor-1,3-, 5 1-Chlor-2,4-dinitrobenzol, o-, m-, p-Chlornitrobenzol, 1, 2-Dichlor-4-, 1,4-Dichlor-2-, 2,4-Dichlor-1-und 1,2-Dichlor-3-nitrobenzol, Chlomitrotoluole, wie z.B. 4-Chlor-2, 4-Chlor-3-, 2-Chlor-4- und 2-Chlor-6-nitrotoluol, Nitroaniline, wie z.B. o-, m-, p- Nitroanilin; Nitroalkohole, wie z.B. 10 Tris(hydroxymethyl)nitromethan, 2-Nitro-2-methyl-, 2-Nitro-2-ethyl-1,3-propandiol, 2-Nitro-1-butanol und 2-Nitro-2-methyl-1-propanol sowie beliebige Gemische aus zwei oder mehreren der genannten Nitroverbindungen eingesetzt.

Bevorzugt werden nach dem erfindungsgemäßen Verfahren aromatische Nitroverbindungen, vorzugsweise Mononitrobenzol, Methylnitrobenzol oder Methylnicrotoluol, und insbesondere 2,4-Dinitrotoluol oder dessen technische Gemische mit 2,6-Dinitrotoluol, wobei diese Gemische vorzugsweise bis zu 35 Gewichtsprozent, bezogen auf das Gesamtgemisch, an 2,6-Dinitrotoluol mit Anteilen von 1 bis 4 Prozent an vicinalem DNT und 0,5 bis 1,5 % an 2,5- und 3,5-Dinitrotoluol aufweisen, zu den entsprechenden Aminen hydriert.

Insbesondere bei der Hydrierung von Dinitrotoluolisomeren zu den entsprechenden Toluylendiaminderivaten (TDA) und bei der Hydrierung von Mononitrobenzol zu Anilin kann das erfindungsgemäße Verfahren vorteilhaft eingesetzt werden. Die Bildung von hochmolekularen, teerartigen Nebenprodukten, die bei den Verfahren des Standes der Technik zu Ausbeuteverlusten sowie zum Verkleben und damit zur vorzeitigen Desaktivierung des Katalysators führte, konnte praktisch vollständig unterdrückt werden. Damit ist auch die Reinigung der gebildeten aromatischen Amine, insbesondere des TDA, unkomplizierter als bei den Verfahren des Standes der Tech-nik.

Die Hydrierung des Dinitrotoluols und des Mononitrobenzols kann in Lösung durchgeführt werden. Als Lösungsmittel werden die dafür üblichen Stoffe, insbesondere niedere Alkohole, vorzugsweise Ethanol, eingesetzt. Aufgrund der optimalen Strömungsverhältnisse und der sofortigen Abfuhr der Reaktionswärme in dem erfindungsgemäß verwendeten Reaktor ist es möglich, die Hydrierung auch ohne Lösungsmittel durchzuführen. Das hat die Vorteile, daß das Volumen der Reaktionsmischung geringer ist, was eine kleinere Dimensionierung des Reaktors sowie der Pumpen und Rohrleitungen ermöglicht, daß Nebenreaktionen zwischen dem Lösungsmittel und den Edukten ausgeschlossen werden sowie der Aufwand für die Aufarbeitung der Endprodukte verringert wird.

Zur Durchführung des erfindungsgemäßen Verfahrens werden die an sich bekannten Hydrierkatalysatoren für aromatische Nitroverbindungen verwendet. Es können homogene und/oder insbesondere heterogene Katalysatoren eingesetzt werden. Die heterogenen Katalysatoren werden in feinverteiltem Zustand eingesetzt und liegen in der Reaktionssuspension feinteilig suspendiert vor. Geeignete Katalysatoren sind Metalle der VIII. Nebengruppe des Periodensystems, die auf Trägermaterialien wie Aktivkohle oder Oxiden des Aluminiums, des Siliciums oder anderer Materialien aufgebracht sein können. Vorzugsweise werden Raney-Nickel und/oder geträgerte Katalysatoren auf Basis von Nickel, Palladium und/oder Platin verwendet.

In einer besonders vorteilhaften Ausführungsform des erfindungsgemäßen Verfahrens zur Herstellung der aromatischen Amine wird als Katalysator ein mit Kohlenstoff geträgerter Platin/Eisen-Katalysator verwendet. Als Kohlenstoffträger wird insbesondere ein hydrophober Kohlenstoff eingesetzt. Vorzugsweise kommen Aktivkohle oder Acetylenruß zur Anwendung. Das Platin liegt auf dem Träger üblicherweise als Metall vor. Das Eisen liegt üblicherweise als Eisenverbindung, vorzugsweise als Eisen-(III)-oxid, vor. Das Platin liegt zumeist in einer Menge im Bereich von 0,1 bis 10 Gew.-%, bezogen auf das Gewicht des Trägers, und das Eisen in einer Menge im Bereich zwischen 0,1 bis 10 Gew.-%, berechnet als Eisen-(III)-oxid und ebenfalls bezogen auf den Träger, vor.

Die Herstellung des Katalysators erfolgt nach üblichen und bekannten Verfahren, wie sie beispielsweise in US-A-2,823,235 beschrieben sind.

Überraschenderweise hat sich gezeigt, daß bei Verwendung des bevorzugten Katalysators zur Durchführung des erfindungsgemäßen Verfahrens besonders hohe Raum-Zeit-Ausbeuten und eine besonders hohe Selektivität der Hydrierung, insbesondere eine weitgehende Unterdrückung der Kernhydrierung, erreicht werden können. So konnte mit dem bevorzugt verwendeten Katalysator bei Durchführung der Hydrierung von Mononitrobenzol in einem erfindungsgemäßen Reaktor bei einer Reaktionstemperatur von 180°C eine Raum-Zeit-Ausbeute von 2300 kg Anilin/(m^{3*}h) bei einer Selektivität von 99,9 erreicht werden, während bei gleichen Bedingungen und Verwendung eines auf Zeolith geträgerten Nickelkatalysators nur eine Raum-Zeit-Ausbeute von 1500 kg Anilin/(m^{3*}h) und eine Selektivität von unter 98 % erzielt wurden. Weiterhin ist die Desaktivierung des bevorzugt eingesetzten Katalysators im Vergleich mit anderen Katalysatoren deutlich geringer. So konnten mit 1g eines erfindungsgemäß bevorzugten Katalysators 10 kg Anilin hergestellt werden, während bei einem Zeolith geträgerten Nickelkatalysator nur 0,8 kg Anilin hergestellt werden konnten.

Durch die Dispergierung der einzelnen Reaktanden wird in Verbindung mit den übrigen Reaktionsparametern eine intensive Durchmischung aller Komponenten bei niedrigen Substratkonzentrationen, hohen Stoffübergangskoeffizienten und hohen volumenspezifischen Phasengrenzflächen erreicht. Die Anordnung der Kühlrohre im Reaktor parallel zu den Reaktorwänden hat eine nahezu vollständige Gradientenfreiheit des Reaktorinhalts bezüglich der Reaktionstemperatur zur Folge. Durch Vermeidung örtlicher Überhitzungen wer-den Nebenreaktionen deutlich zurückgedrängt und Katalysatordesaktivierung weitgehend vermieden. Es werden damit selbst bei niedrigen Katalysatorkonzentrationen hohe Raum-Zeit-Ausbeuten bei hoher Selektivität erreicht. Aufgrund des Vorhandenseins eines äußeren Umpumpkreislaufs und der Mischungsverhältnisse im Reaktor hat der Schlaufenreaktor bezüglich seines Verweilzeitverhaltens die Charakteristik eines Rührkessels.

Wie oben beschrieben, ist das erfindungsgemäße Verfahren besonders geeignet für die Hydrierung von Dinitrotoluol zu Toluylen-diamin. Gerade bei dieser Reaktion kommt es bei den Verfahren des Standes der Technik zu ausgeprägten Nebenreaktionen mit der Bildung von teerartigen Bestandteilen. Die Herstellung von Toluylendiamin nach dem erfindungsgemäßen Verfahren erfolgt bei den für die Herstellung von aromatischen Aminen üblichen, oben aufgeführten Temperaturen und Drücken. Das Verhältnis von eingespeistem Dinitrotoluol zur äußeren Schlaufenströmung liegt vorzugsweise im Bereich von 1 t Dinitrotouol pro 50 m³ Reaktionsmischung im äußeren Kreislauf bis 1 t Dinitrotoluol pro 60 m³ Reaktionsmischung im äußeren Kreislauf. Diese Menge Dinitrotouol wird der Reaktionsmischung an beliebiger Stelle zugesetzt. Vorzugsweise erfolgt die Einspeisung des Dinitrotoluol in die äußere Schlaufenströmung, besonders bevorzugt in oder kurz vor der Düse.

Die Entnahme des Touylendiamins kann ebenfalls an beliebiger Stelle erfolgen. Insbesondere erfolgt die Entnahme aus der externen Schlaufenströmung vor der Einspeisung des Dinitrotouols. Da die Hydrierung des Dinitrotoluols unter den genannten Bedingung in der internen Schlaufenströmung praktisch quantitativ abläuft, enthält die externe Schlaufenströmung vor der Einspeisung des Dinitrotouols im wesentlichen reines Toluylendiamin, Wasser, gegebenenfalls Lösungsmittel und Katalysator. Das Toluylendiamin wird aus dem abgezogenen Strom abgetrennt und der Reinigung zugeführt, der Katalysator und gegebenenfalls Wasser werden der externen Schlaufenströmung wieder zugeführt.

Auf Grund der geringen Konzentration des Dinitrotoluols in der Reaktionsmischung kann es nicht zur Ausbildung explosiver Gemische kommen.

Die Hydrierung des Dinitrotoluols wird vorzugsweise bei Temperaturen von 80 bis 200 °C und Drücken im Bereich von 10 bis 50 bar durchgeführt.

Das nach dem erfindungsgemäßen Verfahren hergestellte Toluylendiamin ist sehr rein. Die Ausbildung teerartiger Bestandteile kann nahezu völlig unterdrückt werden. Zumeist liegt deren Anteil deutlich unter 1 Gew.-%, bezogen auf das Toluylendiamin. Die Raum-Zeit-Ausbeute bei dem erfindungsgemäßen Verfahren liegt bei über 500g/1/h.

Die Erfindung soll an dem nachfolgenden Beispiel näher erläutert werden.

### Beispiel 1

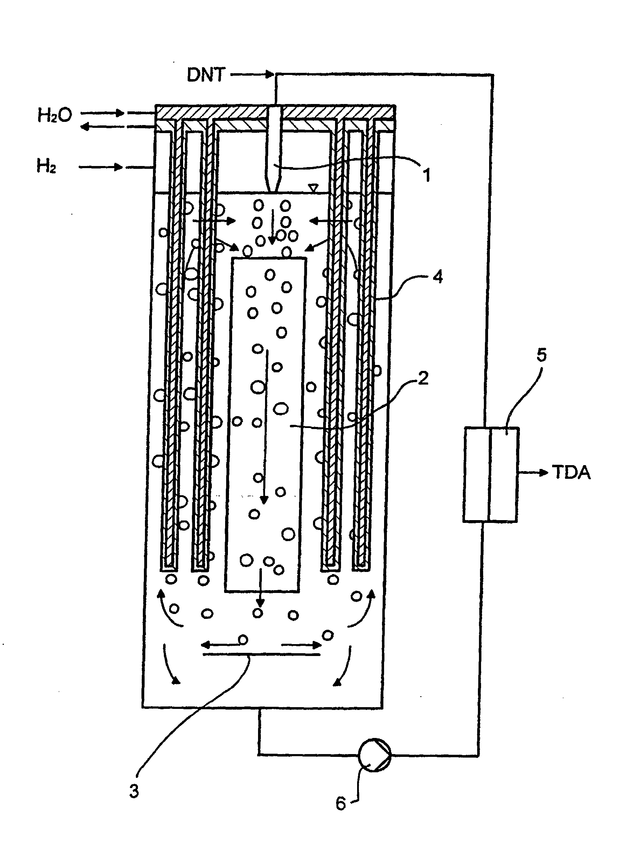

Es wird ein zylinderförmiger Reaktor mit einem außenliegenden Kreislauf, einer Prallplatte im unteren Reaktorteil sowie einem konzentrischen Einsteckrohr eingesetzt, wie er in der Abbildung dargestellt ,ist. Das Reaktionsvolumen des Reaktors beträgt rund 0,05 m³. Der Reaktor ist mit 36 parallel geschalteten Fieldrohren versehen, die insgesamt einer Kühlfläche von etwa 2,5 m² entsprechen. Die Menge des in die Fieldrohre eingespeisten Kühlwassers betrug 1 m³/h, die Temperatur des in die Fieldrohre eingespeisten Kühlwassers lag bei 30 °C, die Austrittstemperatur des Kühlwassers betrug 90°C.

Mit einer Hochdruckpumpe wurden 40,3 kg/h einer Dinitrotoluolschmelze. bestehend aus 80 Gewichtsteilen 2,4-Dinitrotoluol und 20 Teilen 2,6-Dinitrotoluol, bei 120 °C in ein schnell fließendes Gemisch aus ca. 62 Gewichtsteilen eines entsprechenden Diaminotoluolgemisches, 36 Gewichtsteilen Wasser und 2 Gewichtsteilen eines feinverteilten Ni-Hydrierkatalysators eingedüst. Durch gleichzeitiges Einleiten von 30 Nm³/h (wo sind Normkubikmeter definiert?) Wasserstoff wurde im Reaktor ein Druck von 25 bar aufrechterhalten. Zur Aufrechterhaltung der Schlaufenströmung wurde im externen Produktkreislauf ein Volumenstrom von 2,6 m³/h umgewälzt. In der Reaktionsdüse herrschte ein Druck von rund 3 bar, der Leistungseintrag betrug 5 kW/m³. Die Reaktion verlief unter nahezu isothermen Bedingungen, da die entstehende Reaktionswärme bereits am Ort ihrer Entstehung abgeführt wurde. Die maximale Reaktionstemperatur im unteren Drittel des Reaktors betrug 122 °C. Dem Reaktor wurden gleichzeitig unter Rückhaltung des Katalysators 26,7 kg/h eines entsprechenden Diaminotoluol-gemisches sowie 15,8 kg/h Wasser kontinuierlich entnommen, was einer Raum-Zeit-Ausbeute von 580 kg Amingemisch/m^{3*}h entsprach. Die Ausbeute an Diamin betrug, bezogen auf eingesetztes Dinitrötoluol, > 99 %. Bei der destillativen Aufarbeitung fielen 0,15 % niedrigsiedende Nebenprodukte ("Leichtsieder") und 0,75 % teerartige Produkte ("Hochsieder") an. Der Gehalt an Nitro- bzw. Aminonitroverbindungen im Produktaustrag lag unterhalb der Nachweisgrenze von 10 ppm. Eine merkliche Desaktivierung des eingesetzten Hydrierkontakts konnte für den oben beschriebenen Betriebszustand auch nach 100 h Reaktionszeit nicht festgestellt werden.

### Beispiel 2

Es wurde ein zylinderförmiger Reaktor mit einem außenliegenden Kreislauf, einer Prallplatte im unteren Reaktorteil sowie einem konzentrischen Einsteckrohr eingesetzt, wie er in der Abbildung dargestellt ist. Das Reaktionsvolumen des Reaktors beträgt rund 0,05 m³. Der Reaktor ist mit 36 parallel geschalteten Fieldrohren versehen, die insgesamt einer Kühlfläche von etwa 2,5 m² entsprechen. Die Menge des in die Fieldrohre eingespeisten Kühlwassers betrug 2,5 m³/h, die Temperatur des in die Fieldrohre eingespeisten Kühlwassers lag bei 30°C. Die Austrittstemperatur des Kühlwassers betrug ca. 90°C.

Mit einer Hochdruckpumpe wurden 134 kg/h flüssigen Mononitrobenzols bei 180°C in ein schnell fließendes Gemisch aus ca. 71 Gewichtsteilen Anilin, 27 Gewichtsteilen Wasser und 2 Gewichtsteilen eines feinverteilten Pt/Fe/C-Hydrierkatalysators eingedüst. Durch gleichzeitiges Einleiten von 75 Nm³/h Wasserstoff wurde im Reaktor ein Druck von 30 bar aufrechterhalten. Zur Aufrechterhaltung der Schlaufenströmung wurde im externen Produktkreislauf ein Volumenstrom von 2,6 m³/h umgewälzt. In der Reaktionsdüse herrschte ein Druck von rund 3 bar, der Leistungseintrag betrug 5 kW/m³. Die Reaktion verlief unter nahezu isothermen Bedingungen, da die entstehende Reaktionswärme bereits am Ort ihrer Entstehung abgeführt wurde. Die maximale Reaktionstemperatur im unteren Drittel des Reaktors betrug 182°C. Dem Reaktor wurden gleichzeitig unter Rückhaltung des Katalysators 101,3 kg/h Anilin sowie 39,2 kg/h Wasser kontinuierlich entnommen, was einer Raum-Zeit-Ausbeute von 2030 kg Anilin/m^{3*}h entsprach. Die Ausbeute an Anilin betrug, bezogen auf das eingesetzte Mononitrobenzol, > 99,5 %. Bei der destillativen Aufarbeitung fielen als Nebenprodukte Cyclohexylamine und N-Cyclohexylamine in einem Konzentrationsbereich von einigen hundert und bis zu tausend ppm an. Die Konzentrationen der zudem als Nebenprodukte anfallenden Cyclohexanole und Cyclohexanone waren vergleichsweise niedriger. Der Gehalt an Mononitrobenzol im Produktaustrag lag unterhalb der Nachweisgrenze von 10 ppm. Eine Desaktivierung des eingesetzten Hydrierkontakts konnte für den oben beschriebenen Betriebszustand nach einer Reaktionszeit von 30 h nicht festgestellt werden.

## Patentansprüche

1. Verfahren zur Herstellung von Aminen durch Hydrierung von Nitroverbindungen, **dadurch gekennzeichnet, daß** die Hydrierung in einem vertikalen Reaktor, dessen Länge größer ist als sein Durchmesser, mit einer im oberen Bereich des Reaktors angeordneten, nach unten gerichteten strahldüse (1), über die die Edukte sowie das Reaktionsgemisch zugeführt werden sowie mit einem Abzug an einer beliebigen Stelle des Reaktors, über den das Reaktionsgemisch in einem äußeren Kreislauf mittels eines Förderorgans (6) der Strahldüse wieder zugeführt wird, sowie einer Strömungsumkehr im unteren Bereich des Reaktors durchgeführt wird und im Reaktor integrierte Wärmetauscher (4) angebracht sind.

2. Verfahren nach Anspruch 1, **dadurch gekennzeichnet, daß** sich der Abzug am unteren Ende des Reaktors befindet.

3. Verfahren nach Anspruch 1, **dadurch gekennzeichnet, daß** sich im unteren Teil des Reaktors eine senkrecht zur Reaktorwand angeordnete Prallplatte (3) befindet.

4. Verfahren nach Anspruch 1, **dadurch gekennzeichnet, daß** im Reaktor ein konzentrisches Einsteckrohr (2) parallel zur Reaktorwand angebracht ist.

5. Verfahren nach Anspruch 1, **dadurch gekennzeichnet, daß** als Wärmetauscher (4) Kühlrohre, Plattenwärmetauscher und/oder Siederohre eingesetzt werden.

6. Verfahren nach Anspruch 1, **dadurch gekennzeichnet, daß** das Verhältnis der im Reaktor zirkulierenden Reaktionsmischung zu der umgepumpten Reaktionsmischung 2:1 bis 30:1 beträgt.

7. Verfahren nach Anspruch 1, **dadurch gekennzeichnet, daß** das Verhältnis der im Reaktor zirkulierenden Reaktionsmischung zu der umgepumpten Reaktionsmischung 5:1 bis 10:1 beträgt.

8. Verfahren nach Anspruch 1, **dadurch gekennzeichnet, daß** als Nitroverbindungen aromatische Nitroverbindungen eingesetzt werden.

9. Verfahren nach Anspruch 8, **dadurch gekennzeichnet, daß** als Nitroverbindungen Nitrobenzol und/oder Dinitrotoluol eingesetzt werden.

10. Verfahren nach Anspruch 1, **dadurch gekennzeichnet, daß** die Umsetzung in Anwesenheit von Katalysatoren durchgeführt wird.

11. Verfahren nach Anspruch 10, **dadurch gekennzeichnet, daß** als Katalysator zur Hydrierung der aromatischen Nitroverbindungen ein mit Kohlenstoff geträgerter Platin-Eisen-Katalysator eingesetzt wird.

12. Verfahren nach Anspruch 11, **dadurch gekennzeichnet, daß** das Platin in einer Menge im Bereich von 0,1 bis 10 Gew.-%, bezogen auf das Gewicht des Trägers, und das Eisen in einer Menge im Bereich von 0,1 bis 10 Gew.-%, berechnet als Eisen-(III)-oxid und ebenfalls bezogen auf den Träger, vorliegt.

13. Verfahren nach Anspruch 1, **dadurch gekennzeichnet, daß** die Hydrierung der aromatischen Nitroverbindungen bei Temperaturen im Bereich von 80 bis 200 °C durchgeführt wird.

14. Verfahren nach Anspruch 1, **dadurch gekennzeichnet, daß** die Hydrierung der aromatischen Nitroverbindungen bei Drücken im Bereich von 10 bis 50 bar durchgeführt wird.

## Claims

1. A process for the preparation of amines by hydrogenation of nitro compounds, which comprises carrying out the hydrogenation in a vertical reactor whose length is greater than its diameter, having a downward-facing jet nozzle (1) arranged in the upper region of the reactor through which the starting materials and the reaction mixture are fed in, and having an outlet at any desired point of the reactor, through which the reaction mixture is fed back to the jet nozzle in an external circuit by means of a conveying means (6), and having flow reversal in the lower region of the reactor, and integrated heat exchangers (4) installed in the reactor.

2. A process as claimed in claim 1, wherein the outlet is located at the lower end of the reactor.

3. A process as claimed in claim 1, wherein a baffle plate (3) arranged perpendicular to the reactor wall is located in the lower part of the reactor.

4. A process as claimed in claim 1, wherein a concentric spigot (2) is installed in the reactor parallel to the reactor wall.

5. A process as claimed in claim 1, wherein the heat exchangers (4) employed are cooling tubes, plate heat exchangers and/or boiling tubes.

6. A process as claimed in claim 1, wherein the ratio between reaction mixture circulating in the reactor and pumped reaction mixture is from 2:1 to 30:1.

7. A process as claimed in claim 1, wherein the ratio between the reaction mixture circulating in the reactor and the pumped reaction mixture is from 5:1 to 10:1.

8. A process as claimed in claim 1, wherein the nitro compounds employed are aromatic nitro compounds.

9. A process as claimed in claim 8, wherein the nitro compounds employed are nitrobenzene and/or dinitrotoluene

10. A process as claimed in claim 1, wherein the reaction is carried out in the presence of catalysts.

11. A process as claimed in claim 10, wherein the catalyst employed for the hydrogenation of the aromatic nitro compounds is a platinum/iron catalyst supported on carbon.

12. A process as claimed in claim 11, wherein the platinum is present in an amount from 0.1 to 10% by weight, based on the weight of the support, and the iron is present in an amount in the range from 0.1 to 10% by weight, calculated as iron(III) oxide and likewise based on the support.

13. A process as claimed in claim 1, wherein the hydrogenation of the aromatic nitro compounds is carried out in the range from 80 to 200°C.

14. A process as claimed in claim 1, wherein the hydrogenation of the aromatic nitro compounds is carried out at from 10 to 50 bar.

## Revendications

1. Procédé pour la préparation d'amines par hydrogénation de composés nitro, **caractérisé en ce qu'**on effectue l'hydrogénation dans un réacteur vertical dont la longueur est supérieure à son diamètre, comprenant un éjecteur (1) orienté vers le bas et disposé dans la zone supérieure du réacteur, à travers lequel sont acheminés les éduits, ainsi que le mélange réactionnel, et comprenant une évacuation, prévue à n'importe quel endroit du réacteur, par laquelle le mélange réactionnel peut être à nouveau acheminé à l'éjecteur dans un circuit externe à l'aide d'un organe de transport (6), ainsi qu'une inversion du courant dans la zone inférieure du réacteur, des échangeurs de chaleur (4) étant montés à l'état intégré dans le réacteur.

2. Procédé selon la revendication 1, **caractérisé en ce que** l'évacuation se trouve à l'extrémité inférieure du réacteur.

3. Procédé selon la revendication 1, **caractérisé en ce qu'**une plaque de rebondissement (3), disposée perpendiculairement à la paroi du réacteur, se trouve dans la partie inférieure du réacteur.

4. Procédé selon la revendication 1, **caractérisé en ce qu'**un tube d'insertion concentrique (2) est monté dans le réacteur, parallèlement à la paroi du réacteur.

5. Procédé selon la revendication 1, **caractérisé en ce qu'**on met en oeuvre, à titre d'échangeurs de chaleur (4), des tubes de refroidissement, des échangeurs de chaleur à plaque et/ou des tubes de chaudières.

6. Procédé selon la revendication 1, **caractérisé en ce que** le rapport du mélange réactionnel en circulation dans le réacteur au mélange réactionnel transvasé par pompage s'élève de 2 : 1 à 30: 1.

7. Procédé selon la revendication 1, **caractérisé en ce que** le rapport du mélange réactionnel en circulation dans le réacteur au mélange réactionnel transvasé par pompage s'élève de 5 : 1 à 10:1.

8. Procédé selon la revendication 1, **caractérisé en ce qu'**on met en oeuvre, à titre de composés nitro, des composés nitro aromatiques.

9. Procédé selon la revendication 8, **caractérisé en ce qu'**on met en oeuvre à titre de composés nitro, du nitrobenzène et/ou du dinitrotoluène.

10. Procédé selon la revendication 1, **caractérisé en ce qu'**on effectue la mise en réaction en présence de catalyseurs.

11. Procédé selon la revendication 10, **caractérisé en ce qu'**on met en oeuvre, à titre de catalyseur pour l'hydrogénation des composés nitro aromatiques, un catalyseur à base de platine-fer déposé sur un support en carbone.

12. Procédé selon la revendication 11, **caractérisé en ce que** le platine est présent en une quantité dans la plage de 0,1 à 10 % en poids, rapportés au poids du support, et le fer est présent en une quantité dans la plage de 0,1 à 10 % en poids, calculés comme oxyde de fer(lll) et également rapportés au support.

13. Procédé selon la revendication 1, **caractérisé en ce qu'**on effectue l'hydrogénation des composés nitro aromatiques à des températures dans la plage de 80 à 200 °C.

14. Procédé selon la revendication 1, **caractérisé en ce qu'**on effectue l'hydrogénation des composés nitro aromatiques sous des pressions dans la plage de 10 à 50 bar.
